(19) Europäisches Patentamt / European Patent Office / Office européen des brevets

(11) **EP 3 912 609 B1**

(12) **EUROPEAN PATENT SPECIFICATION**

(45) Date of publication and mention
of the grant of the patent:
**17.04.2024  Bulletin 2024/16**

(51) International Patent Classification (IPC):
**A61G 7/05** *(2006.01)*       **G16H 40/63** *(2018.01)*
**G16H 50/30** *(2018.01)*

(21) Application number: **21170456.4**

(22) Date of filing: **26.04.2021**

(52) Cooperative Patent Classification (CPC):
**A61G 7/05; G16H 40/63; G16H 50/30;**
A61G 2203/32

(54) **MOBILITY SENSING USING LOAD CELLS**

MOBILITÄTSERFASSUNG UNTER VERWENDUNG VON KRAFTMESSZELLEN

DÉTECTION DE MOBILITÉ À L'AIDE DE CELLULES DE CHARGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **30.04.2020   US 202063017790 P**

(43) Date of publication of application:
**24.11.2021   Bulletin 2021/47**

(73) Proprietor: **Hill-Rom Services, Inc.**
**Batesville, IN 47006-9167 (US)**

(72) Inventors:
• **Receveur, Timothy J.**
**Batesville 47006-9167 (US)**

• **Embree, Stephen R.**
**Batesville 47006-9167 (US)**
• **Wiggermann, Neal**
**Batesville 47006-9167 (US)**
• **Knouse, William J.**
**Batesville 47006-9167 (US)**

(74) Representative: **Findlay, Alice Rosemary**
**Reddie & Grose LLP**
**The White Chapel Building**
**10 Whitechapel High Street**
**London E1 8QS (GB)**

(56) References cited:
**WO-A1-2016/073186**

**Description**

The present disclosure is related to patient support apparatuses that monitor patient conditions. More specifically, the present disclosure is related to a patient support apparatus that includes a control system that monitors movement of a patient supported on the patient support apparatus and displays information related to the movement on a user interface.

The mobility of a person supported on a patient support apparatus is of interest to caregivers in assessing the risk of the patient developing skin injuries, such as pressure sores, for example. Generally, mobility is scored subjectively by caregivers. Many factors introduce error into the mobility scoring process.

WO2016073186A1 discloses a person support apparatus comprising: a support deck adapted to support thereon an occupant of the person support apparatus, the support deck comprising a first section and a second section, the first section being pivotable with respect to the second section about a generally horizontal pivot axis; a plurality of force sensors adapted to detect a load, the load including the support deck and the occupant when the occupant is supported on the deck; an angle sensor adapted to determine an angle of the first section with respect to a reference; and a controller adapted to issue an alert if outputs from the plurality of force sensors meet at least one criterion, the controller taking into account the angle when determining whether to issue the alert or not. The criterion may be a center of gravity of the load moving outside of a defined area. The controller may detect a change in the center of gravity of the load and estimates, using the angle, what portion of the change is due to movement of the occupant relative to the support deck and what portion of the change is due to pivoting of the first section.

The present invention is disclosed in the appended claims.

According to the present invention, a patient support apparatus includes a frame and a patient support surface positioned on the frame, a plurality of force sensors coupled to at least one of the frame and the patient support surface, each of the plurality of force sensors configured to detect a force applied to the respective force sensor, and a controller in communication with each of the plurality of force sensors, the controller comprising a processor and a memory device, wherein the memory device includes instructions that, when executed by the processor, cause the processor to process data signals from the plurality of force sensors to determine a mobility score of a patient supported on the patient support surface, wherein determining the mobility score includes executing the instructions with the processor to cause the processor to: determine a change of position of a center of gravity of the patient, multiply the change of position of the center of gravity by a patient weight to compute an instantaneous work, sum the instantaneous work over a time period to compute a power, and utilize the power as an input to a statistical model to generate the mobility score.

In some embodiments of the first aspect, the mobility score may be output to a user. The mobility score may be output to a display of a user interface. The data signals from the plurality of force sensors may be processed in real-time.

Optionally, in the first aspect, the memory device may include instructions that, when executed by the processor, cause the mobility score to be stored over time. The memory device may include instructions that, when executed by the processor, create a data array of the mobility score stored overtime. The memory device may include instructions that, when executed by the processor, cause at least a portion of the data array to be displayed as a graph. The memory device may include instructions that, when executed by the processor, display the graph on a display of a user interface.

It may be desired, in the first aspect, that the mobility score may include a plurality of mobility indices. Each of the mobility indices may be modified by at least one predetermined factor. The at least one predetermined factor may include at least one of a patient factor, a bed factor, and a statistical scaling factor.

It may be contemplated, in the first aspect, that the mobility score over time may include a number of data points. Each of the data points may be selectable on the user interface to display additional information related to the respective data point. The additional information may include a summary of data accumulated to a point in time corresponding with the data point. The additional information may be displayed on a display of a user interface. The additional information may include a graphical indication of a time at which the patient moved relative to the patient support surface.

According to a second aspect of the disclosed embodiments, a patient support apparatus may include a frame. A patient support surface may be positioned on the frame and may include a plurality of bladders organized into a plurality of zones. Each of a plurality of pressure sensors may be operable to measure a pressure in a respective zone. A plurality of load cells may be coupled to the frame. A controller may be in communication with each of the plurality of pressure sensors to receive data signals indicative of a pressure applied to each of the plurality of pressure sensors. The controller may also be in communication with the load cells to receive data signals indicative of a force applied to each of the plurality of load cells. The controller may include a processor and a memory device. The memory device may include instructions that, when executed by the processor, process the data signals from the plurality of pressure sensors and the data signals from the plurality of load cells to determine if the data signals from the plurality of pressure sensors and the data signals from the plurality of load cells are related to a patient on the patient support surface or a load independent of the patient.

In some embodiments of the second aspect, the memory device may include instructions that, when executed by the processor, output an indication of a cause of the data signals from the plurality of pressure sensors and the data signals from the plurality of load cells. The indication may be an indication that the data signals from the plurality of pressure sensors and the data signals from the plurality of load cells are related to the patient. The indication may be an indication that the data signals from the plurality of pressure sensors and the data signals from the plurality of load cells are related to a load independent of the patient.

Optionally, in the second aspect, the controller may receive the data signals from the plurality of pressure sensors and the data signals from the plurality of load cells simultaneously. The controller may be in communication with a user interface and operable to cause the user interface to display at least one of the data signals from the plurality of pressure sensors and the data signals from the plurality of load cells. The memory device may include instructions that, when executed by the processor, cause the data signals from the plurality of pressure sensors and the data signals from the plurality of load cells to be displayed as a graph.

According to a third aspect of the disclosed embodiments, a patient support apparatus may include a frame. A weigh frame may be coupled to the frame and may include a pair of long edges and a pair of short edges extending between the long edges. A plurality of load cells may be positioned on the weigh frame. Each of the plurality of load cells may be configured to detect a force applied to a respective load cell. A controller may be in communication with the plurality of load cells and configured to receive data signals indicative of the force applied to each respective load cell. The controller may include a processor and a memory device. The memory device may include instructions that, when executed by the processor, process the data signals from each of the plurality of load cells to detect patient movement by comparing data signals from the load cells positioned on the long edges of the weigh frame and the load cells positioned on the short edges.

In some embodiments of the third aspect, the detected patient movement may include lateral patient movement. The detected patient movement may include detecting that the patient is supine or turned. The detected patient movement may include detecting whether a caregiver offloaded a section of the patient support apparatus before a patient turn occurred.

Optionally, in the third aspect, the controller may output an indication of the detected patient movement to a user. The controller may be in communication with the user interface and operable to cause the user interface to display the data signals from each of the plurality of load cells. The memory device may include instructions that, when executed by the processor, cause the data signals from each of the plurality of load cells to be displayed as a graph.

According to a fourth aspect of the disclosed embodiments, a patient support apparatus may include a frame. A patient supporting surface may positioned on the frame and may include a plurality of bladders organized into a plurality of zones. Each of a plurality of pressure sensors may measure a pressure in a respective zone. A weigh frame may be coupled to the frame and may include a plurality of load cells. A controller may be in communication with each of the plurality of pressure sensors to receive data signals indicative of a pressure in each of the plurality of zones. The controller may also be in communication with the plurality of load cells to receive data signals indicative of a force on each load cell. The controller may include a processor and a memory device. The memory device may include instructions that, when executed by the processor detect movement signals from at least one of the data signals indicative of a pressure in each of the plurality of zones and the data signals indicative of a force on each load cell. The movement signals may be processed using a Fourier transform to determine peaks in the movement signals that correlate to an onset of a disease.

In some embodiments, of the fourth aspect, the disease may be Parkinson's disease. The peaks in the movement signals may be in a range of 3 Hz to 6 Hz. The disease may be an epileptic seizure.

Optionally, in the fourth aspect, the controller may be in communication with a user interface and operable to cause the user interface to display data related to the peaks. The memory device may include instructions that, when executed by the processor, cause the peaks detected by the system to be displayed as a graph.

The invention will now be further described by way of example with reference to the accompanying drawings, in which:

Fig. 1 is a side perspective view of a patient support apparatus according to an embodiment;
Fig. 2 is a schematic diagram of a control system of the patient support apparatus shown in Fig. 1;
Fig. 3 is a schematic diagram of a plurality of bladders organized into zones of the patient support apparatus shown in Fig. 1;
Fig. 4 is a schematic diagram of a weigh scale of the patient support apparatus shown in Fig. 1;
Fig. 5 is a schematic diagram of a user interface having a display of the patient support apparatus shown in Fig. 1;
Fig. 6 is a flowchart for a method of determining a patient mobility score in accordance with an embodiment;
Fig. 7 is a graph of patient mobility data that may be displayed in the display shown in Fig. 5;
Fig. 8 is a flowchart of a method of determining patient turns on the patient support apparatus;
Fig. 9 is a graph of patient turn data that may be displayed in the display shown in Fig. 5;
Fig. 10 is a flowchart of a method of determining a source of pressure on the patient support apparatus;
Fig. 11 is a flowchart of a method of detecting patient disease using movement data from the patient support apparatus;

Fig. 12 is a chart of patient mobility data that may be displayed in the display shown in Fig. 5;

Fig. 13 is a graph showing signal traces of sensors of the patient support apparatus of Fig. 1 under a use condition where a patient is moving on the bed; and

Fig. 14 is a graph similar to the graph of Fig. 13, the graph of Fig. 14 showing signal traces of sensors of the patient support apparatus of Fig. 1 under a use condition where an extraneous load is added to the bed.

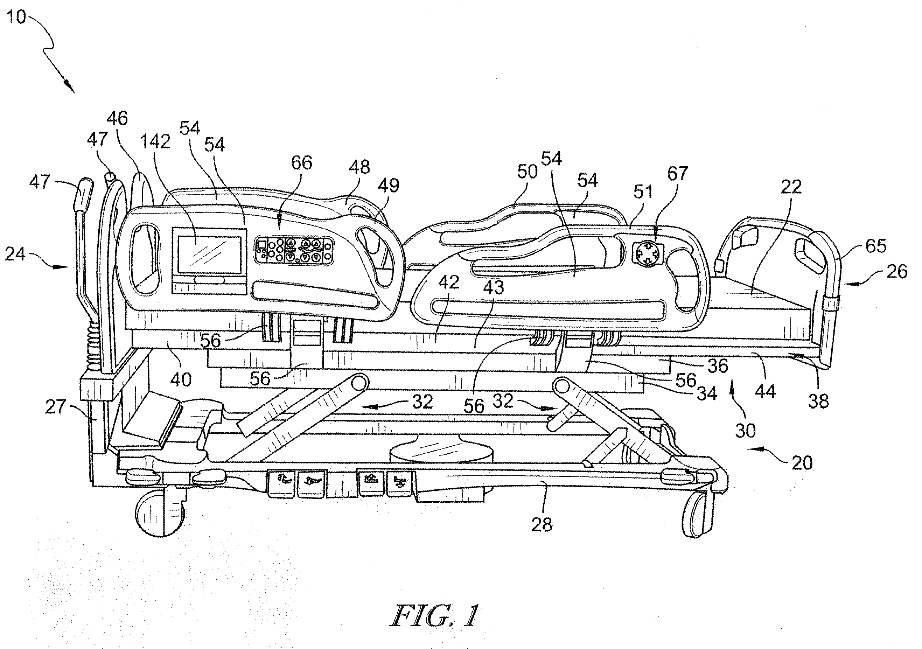

A patient support apparatus 10, illustratively embodied as a hospital bed 10, includes a frame 20 that supports a mattress 22 as shown in Fig. 1. According to this disclosure a bed frame, a mattress or both are examples of structures considered to be within the scope of a term "patient support structure." However, this disclosure is applicable to other types of patient support apparatuses and other patient support structures, including other types of beds, surgical tables, examination tables, stretchers, and the like.

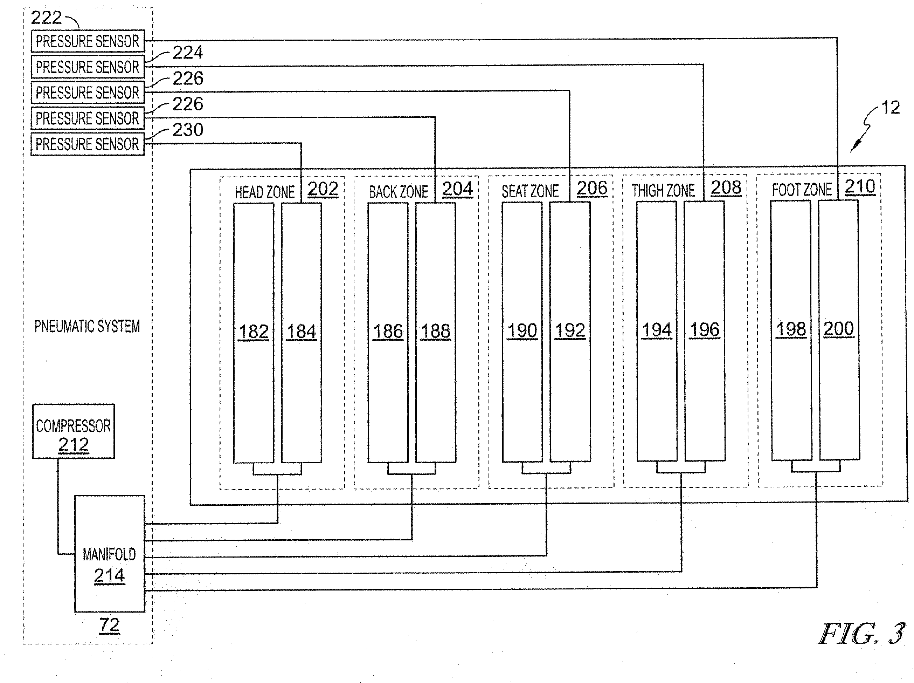

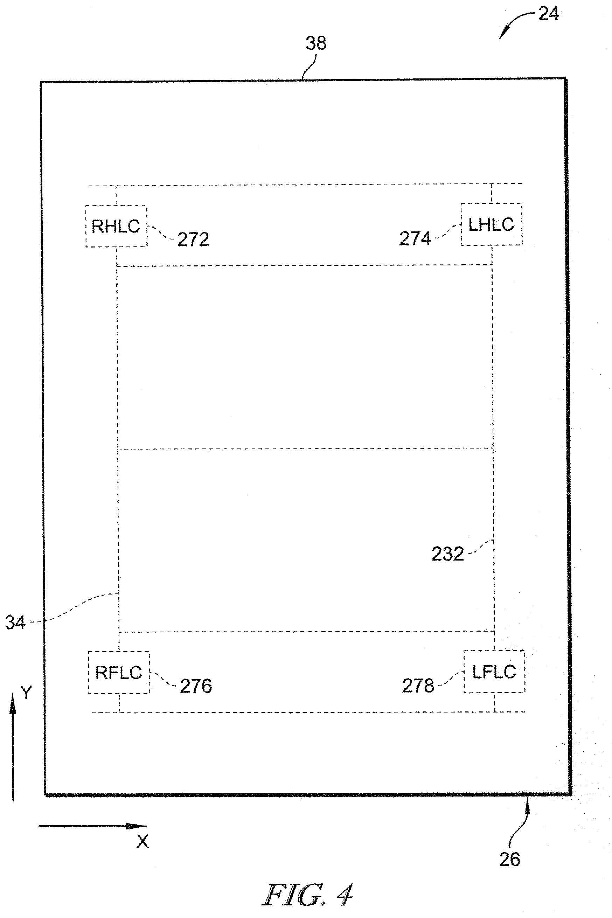

As will be described in further detail below, the present disclosure is directed to the application of sensors (discussed in detail below) of hospital bed 10, or other patient support apparatuses, to detect certain conditions and physiological characteristics of a patient supported on the frame 20 and/or mattress 22. Because a patient spends a majority of their time, when they are in a care facility such as a hospital, for example, in a patient support apparatus such as hospital bed 10, the use of existing sensors on the hospital bed 10 to detect certain characteristics of the patient provides efficiencies. For example, because the sensors are already present in the patient support apparatus 10 and the detection of the sensors occurs without directly engaging the patient, there is no additional cost for separate sensor systems and the patient is less likely to be concerned with or attempt to manipulate the sensors and their output. This ongoing ability to use signal from a set of load cells 272, 274, 276, 278 (see Fig. 4) coupled to portions of the frame 20 and/or pressure sensors 222, 224, 226, 228, 230 (see Fig. 3) of the mattress 22 allows data to be gathered and processed over an extended period to infer certain conditions and physiological characteristics of the patient and to test those inferences once they are made.

Referring again to Fig. 1, the frame 20 of the bed 10 includes a base 28, an upper frame assembly 30, and a lift system 32 coupling the upper frame assembly 30 to the base 28. The lift system 32 is operable to raise, lower, and tilt the upper frame assembly 30 relative to the base 28. For reference to orientation, bed 10 has a head end 24 which is associated with the general direction of a patient's head when a patient is normally positioned on the bed 10 in a supine position. The bed 10 also has a foot end 26 opposite the head end 24.

The upper frame assembly 30 includes an intermediate frame 34, a weigh frame 36 supported with respect to intermediate frame 34, and a patient support deck 38. The patient support deck 38 is carried by the weigh frame 36 and engages the surface 22. As will be discussed in further detail below, the load cells 272, 274, 276, 278 are supported on the intermediate frame and support the weigh frame 36 so that loads are transferred through the load cells 272, 274, 276, 278. The patient support deck 38 includes a head section 40, a seat section 42, a thigh section 43 and a foot section 44. The sections 40, 43, 44 are each movable relative to the weigh frame 36. For example, the head section 40 pivotably raises and lowers relative to seat section 42 whereas the foot section 44 pivotably raises and lowers relative to thigh section 43. Additionally, the thigh section 43 articulates relative to the seat section 42. Also, in some embodiments, the foot section 44 is extendable and retractable to change the overall length of the foot section 44 and therefore, to change the overall length of the deck 38.

Referring to Fig. 4, load cells 272, 274, 276, 278 are part of a weigh scale 270 are positioned between the weigh frame 36 and the intermediate frame 34 on the patient support deck 38. The weight distribution of a load among the plurality of load cells 272, 274, 276, 278, may not be the same depending on sensitivities of each of load cells 272, 274, 276, 278, and a position of a load on the patient support surface 22 that is transferred to the weigh frame 36. For clarity, the diagrammatic representation of Fig. 4 is as if a person is looking down through the deck 38 and the weigh frame 36 is supported on the load cells 272, 274, 276, 278. The load cells 272, 274, 276, 278 are supported on the intermediate frame 34 and support the entire load that is applied to the weigh frame 36 including other components of the frame 20 such as the deck 38, as well as any patient load. A calibration constant for each of the load cells 272, 274, 276, 278, is established to adjust for differences in the load cells 272, 274, 276, 278, in response to the load. An example of methods of calibrating load cells 272, 274, 276, 278 is disclosed in U.S. Pat. No. 10,634,549 titled "Hospital Bed Scale Calibration Methods and Patient Position Monitoring Methods," which integrated by reference herein for the disclosure of methods of load cell calibration. Each of the load cells 272, 274, 276, 278, produces a signal indicative of the load supported by that specific load cell. The loads detected by each of the respective load cells 272, 274, 276, 278, are adjusted using a corresponding calibration constant for the respective load cell 272, 274, 276, 278. The adjusted loads are then combined by control circuitry 98, shown in Fig. 1, to establish the actual weight supported on the load cells 272, 274, 276, 278.

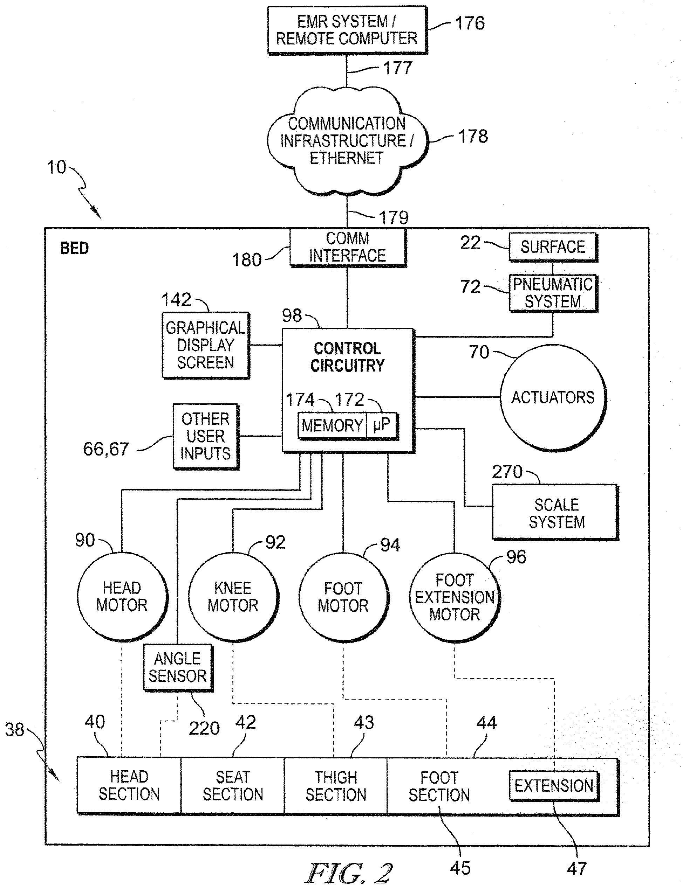

In the illustrative embodiment, the bed 10 includes a pneumatic system 72 (See Figs. 2 and 3) that controls inflation and deflation of various air bladders or cells of the surface 22. Referring now to Fig. 3, the surface 22 includes a number of bladders 182, 184, 186, 188, 190, 192, 194, 196, 198, and 200. The bladders 182, 184, 186, 188, 190, 192, 194, 196, 198, and 200 of the disclosed embodiment are grouped into five zones including a head zone 202, a back zone 204, a seat zone 206, a thigh zone 208, and a foot zone 210 with two bladders in each zone 202, 204, 206, 208, and 210. The bladders for each zone 202, 204, 206, 208, and 210 are in fluid communication with a free flow of air between the bladders. For example, the zone 202 includes the bladders 182 and 184 connected together and in fluid communication. It should be understood that the five zones 202, 204, 206, 208, and 210 are illustrative and that in other embodiments, more a fewer zones and more or fewer bladders may be included.

The pneumatic system 72 includes a source of pressurized air 212, illustratively embodied as a compressor and a manifold 214 that directs air to and from each of the zones 202, 204, 206, 208, and 210. Other sources of pressurized air may be used in other embodiments, such as, for example, a blower, a pump, centralized hospital compressed air, or any other source of pressurized air suitable for inflating the bladders of mattress of a hospital bed. In addition, the pneumatic system 72 includes pressure sensors 222, 224, 226, 228, and 230 that are each in fluid communication with a respective zone 202, 204, 206, 208, and 210 so that the air in the respective zone engages the respective sensor 222, 224, 226, 228, and 230 and operable to measure a pressure in the respective zone. In the illustrative embodiment, the pressure sensors 222, 224, 226, 228, and 230 include a piezoelectric element that provides signals responses to the pressure applied to the piezoelectric element as is known in the art. Referring to Fig. 2, control circuitry 98 is operable to receive a signal indicative of the pressure in each of the zones 202, 204, 206, 208, and 210 from the respective pressure sensors 222, 224, 226, 228, and 230 on a regular basis, such as once every 30 milliseconds, for example. The pressure signal from each sensor 222, 224, 226, 228, and 230 may be collected in an array. The control circuitry 98 is also electrically coupled to the actuators 90, 92, 94, 96 and to the actuators 70 of the lift system 32. The control circuitry 98 is represented diagrammatically as a single block 98 in Fig. 2, but control circuitry 98 in some embodiments comprises various circuit boards, electronics modules, and the like that are electrically and communicatively interconnected. The control circuitry 98 includes one or more processors, microcontrollers, or microprocessors, such as a microprocessor 172 that executes software to perform the various control functions and algorithms described herein. Thus, the control circuitry 98 also includes memory 174 for storing software, variables, calculated values, and the like as is well known in the art. The memory 174 includes instructions, that when executed by the processor(s) cause the control circuitry to perform various algorithms and processes, such as the processes and algorithms disclosed herein.

The processor 172 may be embodied as any type of processor capable of performing the functions described herein. The processor 172 may be embodied as a single or multi-core processor(s), digital signal processor, microcontroller, or other processor or processing/controlling circuit. The memory 174 may be embodied as any type of volatile or nonvolatile memory or data storage capable of performing the functions described herein. In operation, the memory 174 may store various data and software used during operation of the bed 10 such as operating systems, applications, programs, libraries, and drivers. The memory 174 is communicatively coupled to the processor 172 via an I/O subsystem which may be embodied as circuitry and/or components to facilitate input/output operations with the processor 172, the memory 174, and other components of the bed 10. For example, the I/O subsystem may be embodied as, or otherwise include, memory controller hubs, input/output control hubs, integrated sensor hubs, firmware devices, communication links (i.e., point-to-point links, bus links, wires, cables, light guides, printed circuit board traces, etc.) and/or other components and subsystems to facilitate the input/output operations. In some embodiments, the I/O subsystem may form a portion of a system-on-a-chip (SoC) and be incorporated, along with the processor 172, the memory 174, and other components of the bed 10, on a single integrated circuit chip.

A user input block represents the various user inputs such as buttons of control panels 66, 67 shown in Fig. 1, for example, that are used by the caregiver or patient to communicate input signals to the control circuitry 98 of the bed 10 to command the operation of the various actuators 70, 90, 92, 94, 96 of the bed 10, as well as commanding the operation of other functions of the bed 10. The bed 10 includes at least one graphical user input or display screen 142 coupled to the siderail 49. The display screen 142 is coupled to the control circuitry 98. In some embodiments, two graphical display screens 142 are provided and are coupled to the siderail 49. Alternatively or additionally, one or more graphical user interfaces are coupled to the siderail 49 and/or to one or both of the headboard 46 and the footboard 65. It is contemplated by this disclosure that a graphical display screen 142 may be coupled to any of the barriers 65, 46, 48, 49, 50, and/or 51 of bed 10. Alternatively or additionally, the graphical display screen 142 is provided on a hand-held device such as a pod or pendant that communicates via a wired or wireless connection with the control circuitry 98.

The control circuitry 98 receives user input commands from the graphical display screen 142 when the display screen 142 is activated. The user input commands control various functions of the bed 10 such as controlling the pneumatic system 72 and therefore, the surface functions of the surface 22. In some embodiments, the input commands entered on user interface of the display screen 142 also control the functions of one or more of the actuators 70, 90, 92, 94, 96 but this need not be the case. In some embodiments, input commands entered on the user interface of the display screen 142 also control functions of the weigh scale system 270, which is discussed in more detail below.

Illustrative bed 10 has four siderail assemblies 48, 49, 50, 51 coupled to the upper frame assembly 30. The four siderail assemblies 48, 49, 50, 51 include a pair of head siderail assemblies 48, 49 (sometimes referred to as head rails) and a pair of foot siderail assemblies 50, 51 (sometimes referred to as foot rails). Each of the siderail assemblies 48, 49, 50, 51 is movable between a raised position, as shown in Fig. 1, and a lowered position (not shown). Siderail assemblies 48, 49, 50, 51 are sometimes referred to herein as siderails 48, 49, 50, 51. Each siderail 48, 49, 50, 51 includes a barrier panel 54 and a linkage 56. Each linkage 56 is coupled to the upper frame assembly 30 and is configured to guide the barrier panel 54 during movement of siderails 48, 49, 50, 51 between the respective raised and lowered positions. The barrier panel 54 is maintained by the linkage 56 in a substantially vertical orientation during movement of siderails 48, 49, 50, 51 between the respective raised and lowered positions.

Each siderail 49 includes a first user control panel 66 coupled to the outward side of the associated barrier panel 54 and each siderail 51 includes a second user control panel 67 coupled to the outward side of the associated barrier panel 54. The controls panels 66, 67 include various buttons that are used by a caregiver (not shown) to control associated functions of the bed 10. For example, the control panel 66 includes buttons that are used to raise and lower the head section 40, buttons that are used to raise and lower the thigh section 43, and buttons that are used to raise, lower, and tilt the upper frame assembly 30 relative to the base 28. In the illustrative embodiment, the control panel 67 includes buttons that are used to raise and lower foot section 44 and buttons that are used to extend and retract a foot extension 47 relative to main portion 45 (both shown in Fig. 2). In some embodiments, the buttons of control panels 66, 67 comprise membrane switches.

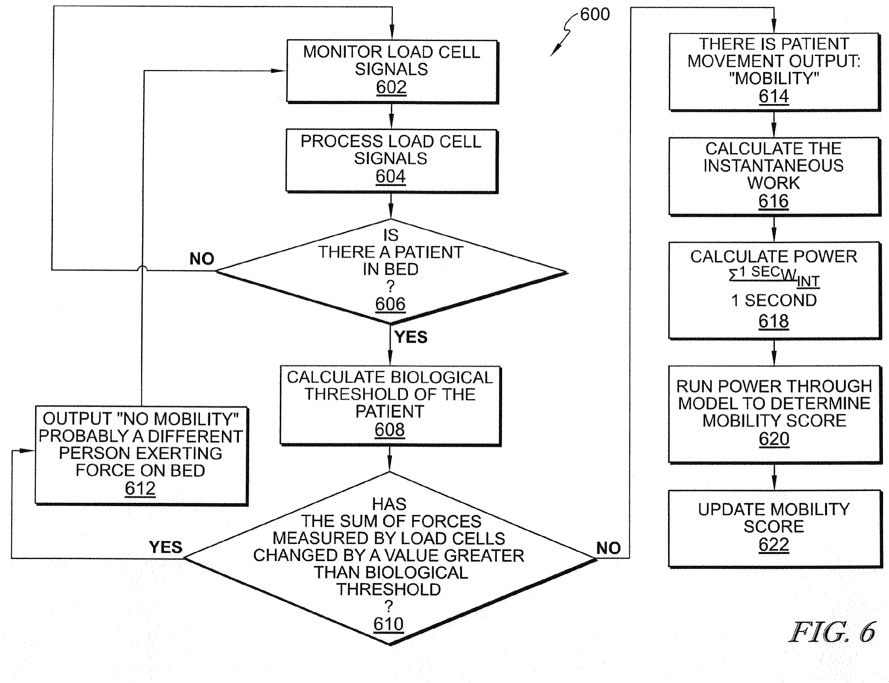

According to one aspect, an approach for determining a value representative of a patient's mobility is disclosed in Fig. 6. The process 600 of Fig. 6 regularly monitors the load cells 272, 274, 276, 278 to determine a patient mobility value or score. It should be understood that the patient mobility value or score could be a numerical value on a pre-defined scale, a qualitative value, or some other expression of the patient's mobility that would provide a meaningful indication to a caregiver of the relative mobility of the patient. This embodiment uses the load cells 272, 274, 276, 278 in the bed 10 to capture the instantaneous work that the patient is doing, which translates into the load cells 272, 274, 276, 278 of the bed 10 and can be used to determine the power exerted by the patient as a measurement of mobility where mobility is defined as the patient's kinetic movements within the confines of the bed 10. In this embodiment, the load cells 272, 274, 276, 278 can be used to capture mobility.

At process step 602, the signals from each load cell 272, 274, 276, 278 is monitored and the process advances. At process step 604, the control circuitry 98 processes the signals from the load cells 272, 274, 276, 278 and stores the values of the signals in memory 174. The loads detected by each of the respective load cells 272, 274, 276, 278 is adjusted using a corresponding calibration constant for the respective load cell 272, 274, 276, 278 as discussed above. The adjusted loads are then combined to establish the actual weight supported on the patient support apparatus 10. At decision step 606, the actual weight is compared to initial weights stored in the memory 174 to determine if a patient is present. If there is no patient present, monitoring of the load cells 272, 274, 276, and 278 is resumed at process step 602 and this loop is repeated until a patient is detected. If a patient is determined to be present, a biological threshold rate change of that patient is calculated at process step 608. Determination of a biological threshold rate is discussed in U.S. Patent No. 10,054,479. At process step 610, the sum of the measurements from the load cells 272, 274, 276, 278 are compared to the biological threshold rate determined at process step 608. If the total weight change is greater than the biological threshold rate, it is determined that data signals being captured are not all a result of changes in weight of the current patient assigned to the patient support apparatus, and the change is due to a different person exerting force on the patient support apparatus or some other extraneous factor not related to patient mobility. This is communicated to the graphical display screen 142 via control circuitry 98 at process step 612, and the monitoring of the load cells at process step 602 is resumed.

If the rate of weight change sensed by the load cells 272, 274, 276, 278 is less than the biological threshold as determined at process step 610, it is determined that patient is moving, and this is communicated to a graphical display screen 142 via control circuitry 98 at process step 614. Furthermore, if the patient is moving as determined in step 614, step 616 is executed. At process step 616, the instantaneous work is calculated as described below.

To determine the instantaneous work, first, the change of position of the center of gravity $\Delta CG_{XY}$ of the patient is calculated using Equation 1 below. This analysis is completed within a two-dimensional Cartesian coordinate system having two horizontally extending X and Y axes along the patient support deck 38 as indicated in Figs. 1 and 4.

$$\Delta CG_{XY} = \sqrt{(\Delta X_{CG})^2 + (\Delta Y_{CG})^2} \tag{1}$$

where:

$$\Delta X_{CG} = X_t - X_{t-1} \tag{2}$$

$$\Delta Y_{CG} = Y_t - Y_{t-1} \tag{3}$$

Then, this change is multiplied by the patient's weight (pw) to determine instantaneous work ($W_{Int}$) in units of force times length.

$$W_{In(t)} = (pw) \times \Delta CG_{XY} \tag{4}$$

In one embodiment, the instantaneous location of the center of gravity is calculated using readings from the load cells 272, 274, 276, 278 at a given time where the reading of a given load cell is designated as $R_i$. The location of the center of gravity is determined using the resolved position of the center of gravity $CG_{xy}$ by determining the constituent positions $X_{CG}$ and $Y_{CG}$ according to equations 5 and 6 below.

$$X_{CG} = \left[ \sum_{i=0}^{3} (R_i \times X_i) \right]/pw \tag{5}$$

$$Y_{CG} = \left[ \sum_{i=0}^{3} (R_i \times Y_i) \right]/pw \tag{6}$$

where:

$$pw = \sum_{i=0}^{3} R_i \tag{7}$$

Power (*P*) over a time period (n) is calculated by summing the instantaneous work of Equation 4 over time according to Equation 8 below.

$$P = \sum_{i=0}^{n} W_{in(i)} \times t_i \tag{8}$$

So, for each time period (n) a value of *P* can be determined to provide a measure of the exertion of a patient over that time. This calculation of the power exerted by a patient is used as an analogue for the mobility of the patient.

At process step 620, the computed power is used as the input to a statistical model to determine a mobility score. At process step 622, the mobility score of the patient is updated in memory 174 and may be communicated to the graphical display screen 142 via the control circuitry 98. A mobility score, such as the mobility score calculated using the approach described above, is used for various clinical analyses and as a predictor of the risk of injury to a patient.

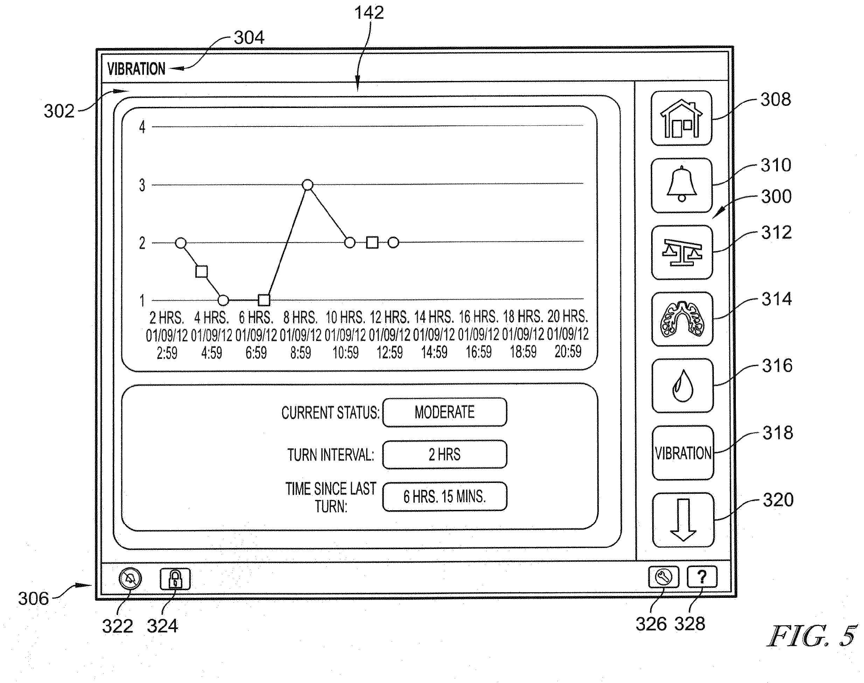

Referring now to Fig. 5, a graphical display screen 142 allows a user to visually review a mobility and mobility history of a person. The graphical display screen 142 includes a navigation bar 300 that includes a number of icons that allow a user to navigate to various functions which may be displayed on the graphical display screen 142. A first display area 302 presents charted data showing the mobility score for a person supported on the bed 10 at a given point in time. The display area 302 also allows a user to navigate through the charted data over time. A status bar 304 along the top of the display 142 provides an indication of what screen is being displayed. In some embodiments, the status bar 304 may also display information about the person supported on the bed 10, such as the patient's name, room number, the current time and date, or other information useful to a caregiver operating the bed 10.

A control bar 306 allows a user to activate an alarm silence icon 322 to silence alarms that may be active. In addition, a screen lock icon 324, when activated, allows a user to lock the display screen 142 so that no inputs will be processed without authorization. For example, a user may be prompted for a password, or the screen may remain locked until the system detects the presence of an authorization token, such as a radio frequency identification transmitter or a similar authorization device. In addition, an icon 326 may be activated by a user to call up a screen displaying maintenance function. An icon 328 may be activated to call up a screen displaying help functionality. In the illustrative embodiment, the control bar 306 is always available to a user regardless of the data or information displayed elsewhere on the graphical display screen 142.

Referring now to the navigation bar 300, a home screen icon 308 allows a user to immediately activate and jump to a home screen (not shown) from any screen display. Similarly, an alarm screen icon 310 will jump to an alarm screen (not shown) from any active screen. The illustrative embodiment of Fig. 4 also includes a scale screen icon 312, a therapy screen icon 314, a moisture screen icon 316, and a mobility screen icon 318. Each of the icons 312, 314, 316, and 318 allow a user to jump to the respective screens for the noted icons. An arrow icon 320 allows a user to move down the navigation bar 300 to display additional navigation icons.

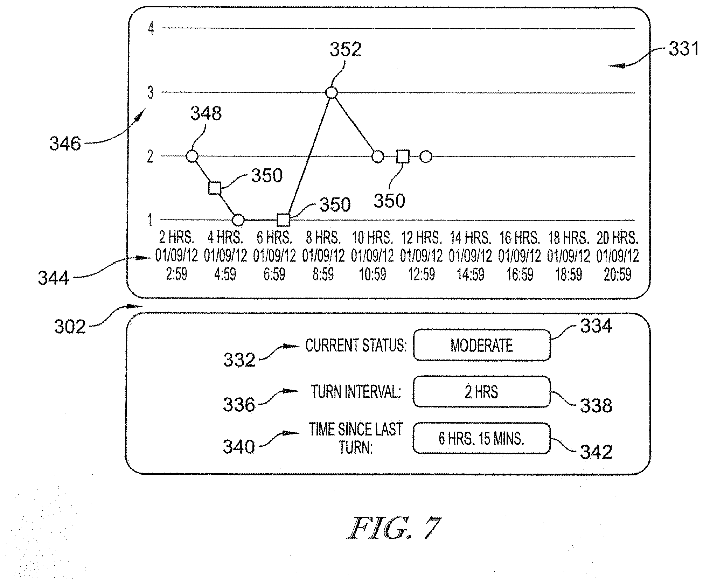

Fig. 7 provides additional details with regard to the mobility screen shown in Fig. 5. In Fig. 7, a graph 331 is displayed on the display area 302 with a time on the horizontal axis 344 and a mobility score value on the vertical axis 346. The graph 331 may be displayed on the graphical display screen 142. A numerical mobility score on a four point scale at a given time is represented by the circular data points such as data points 348 and 352. The icons 350 each represent times of significant movement by the person on the bed 10. Additional information is provided that includes a current status of the mobility of the person with the output 334 displaying a qualitative measure of the person's mobility. A current status 332 includes an output 334 that indicates a degree of patient mobility, for example "moderate." A turn interval indication 336 includes an output 338 that includes an optional turn interval for the patient given their mobility as determined in process 600. The turn interval may be controlled by the therapy functionality available with the therapy screen icon 314, or it may be an indication of the protocol for the person showing how often a nurse or other caregiver should be turning the person as is known in the art. The display area 302 includes an actual turn interval indicator 340 with the time since the last turn displayed at 342.

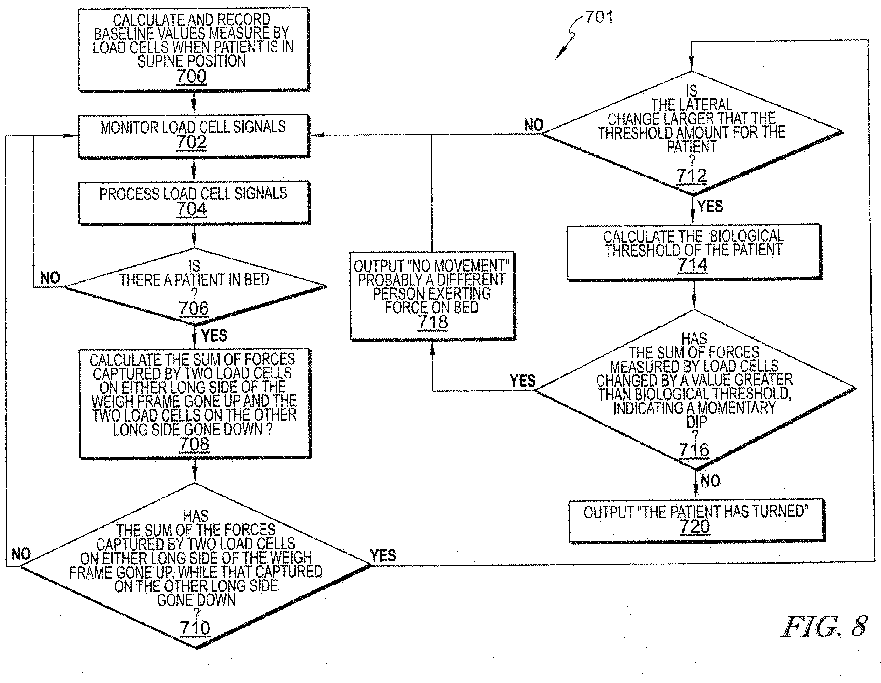

Referring now to Fig. 8, an embodiment of a process 701 to determine if the patient is in a supine position or in a turned position is disclosed. At process step 700, the baseline values measured by the load cells 272, 274, 276, 278, when the patient is in a supine position are determined and recorded for future evaluations. These values are recorded as the threshold values for future measurements. In some embodiments, that may be done as part of an initialization of the scale system 70 of the bed 10 when the patient's weight is first taken. In other embodiments, a caregiver may save the baseline values by activating a button 335 of the display 142 as discussed below.

At process step 702, the signals from each load cell 272, 274, 276, 278 is monitored. At process step 704, the processor module 172 processes the signals from the load cells 272, 274, 276, 278 and stores the signals in memory 174. The loads detected by each of the respective load cells is adjusted using a corresponding calibration constant for the respective load cell as described above. The adjusted loads are then combined to establish the actual weight supported on the patient support apparatus 10 as described with regard to Equation 7 above. At decision step 706, the actual weight is compared to an initial weight stored in the memory 174 to determine if a patient is present. If there is no patient present, step 702 is repeated. If a patient is present, the sum of forces captured by the pairs of load cells 272, 276 or 274, 278 grouped on a long edge of the weigh frame 36, and the sum of forces captured by the load cells 272, 276 or 274, 278 grouped on the other long edge are calculated at process step 708. The sum of forces captured by the load cells on either long edge of the weigh frame 36 is compared to the sum of forces captured by the load cells on the other long edge at decision step 710. If the sum of forces captured by the two load cells grouped on one long edge of the weigh frame 36 shows an increase in value, and the sum of forces captured by the load cells on the other long edge shows a decrease in value, it indicates that the center of gravity of the patient has moved in the two-dimensional Cartesian coordinate system having two horizontally extending X and Y axes along the patient support deck 38.

At decision step 712, it is determined if this lateral change is more than the threshold amount for the patient as calculated at process step 700. If the lateral change is more that the threshold amount, the biological threshold of the patient is calculated at process step 714 as described above with regard to process steps 608 and 610. At decision step 716, the sum of the measurements by all the load cells are compared to the biological threshold determined at process step 714. If the algorithm determines that the weight change is excessive, then the data is ignored for the mobility analysis as being not patient related. This may be communicated to the graphical display screen 144 via control circuitry 98 at process step 718 and the monitoring of the load cells at process step 602 is resumed. If the sum of the load cell measurements is less than the biological threshold as determined at process step 714, it is determined that patient has turned and this may be communicated to a graphical display screen 144 via control circuitry 98 at process step 720. At any given time, the caregiver can invoke the comparison of the values being measured by the force sensors at that time to the baseline values recorded previously, and determines the patient's position.

When tracking the baseline values of all load cells 272, 276 or 274, 278 and, these values may be invoked manually by using a snapshot button 335 by the caregiver on the display 302 as described below. Some other indicators that a patient is supine can be used, such as knowledge that a turn assist has been evoked, then the patient has returned to normal and the patient has been resettled. After the baseline has been captured, the system can keep track if there has been a lateral movement by tracking if the weight measured by load cells on one long edge of the bed has gone up, while the others have gone down. This indicates that the center of gravity has moved by a few inches (wherein 1 inch = 2,54 cm) laterally (in keeping with the anthropocentric data for that patient weight), without directly monitoring the movement of the center of gravity. Additional information, such as momentary dips of the sum of forces on the weigh frame 36, which indicate that a caregiver has offloaded part of the bed before the turn occurred, is also used to determine a turn.

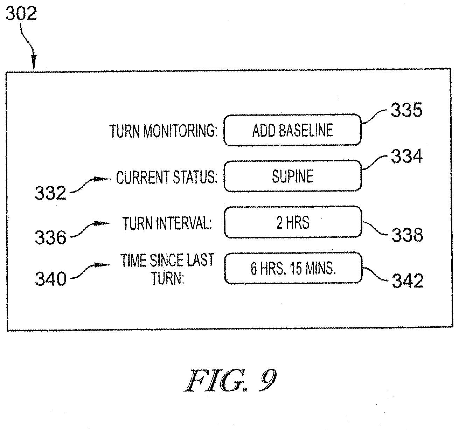

As shown in Fig. 9, the graphical display screen 144 is used to indicate the position of the patient. The display 302 provides information about the current status 332 of the position of the person with the output 334 displaying a measure of the person's position. A turn interval indication 336 includes an output 338 that includes the expected turn interval for the person. The turn interval may be controlled by the therapy functionality available with the therapy screen icon 314, or it may be an indication of the protocol for the person showing how often a nurse or other caregiver should be turning the person as is known in the art. The display 302 also has an actual turn interval indicator 340 with the time since the last turn displayed at 342. The position of the patient in the bed is indicated at 334.

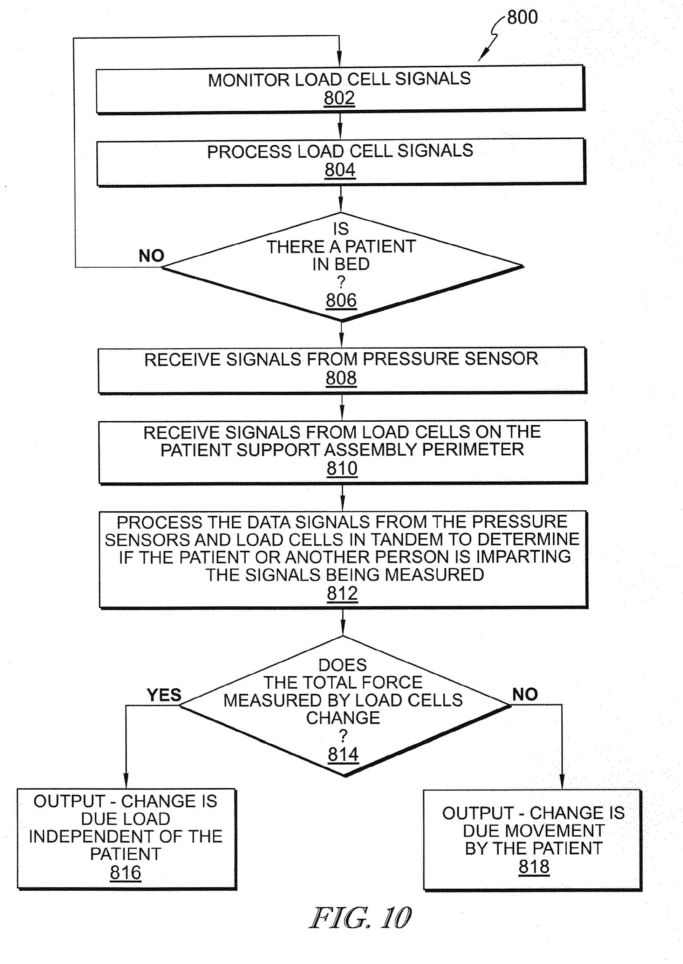

Fig. 10 illustrates yet another process 800 of the present disclosure that uses the data signals captured by the plurality of pressure sensors 222, 224, 226, 228, 230 and the signals captured by the load cells 272, 274, 276, 278 to determine whether loads on the bed are extraneous to the patient. At process step 802, the signal from each load cell 272, 274, 276, 278 is monitored. At process step 804, the processor module 172 processes the signals from the load cells 272, 274, 276, 278 and stores the signals in memory unit 174. The loads detected by each of the respective load cells 272, 274, 276, 278 is adjusted using a corresponding calibration constant for the respective load cell 272, 274, 276, 278. The adjusted loads are then combined to establish the actual weight (pw) supported on the patient support apparatus 10 as described above. At decision step 806, the actual weight is compared to an initial weight stored in the memory 174 to determine if a patient is present. If there is no patient present, step 802 is repeated. If a patient is detected, step 808 is executed. At process step 808, signals from the pressure sensors 222, 224, 226, 228, 230 are received. At process step 810, the signals from the load cells 272, 274, 276, 278 continue to be received. At process step 812, the signals from the pressure sensors 222, 224, 226, 228, 230 and the signals from the load cells 272, 274, 276, 278 are processed in tandem to determine if a non-patient is imparting any of the data signals being captured. The signals are processed to determine the total forces captured by the load cells 272, 274, 276, 278 and the total forces captured by the pressure sensors 222, 224, 226, 228, 230 separately. At decision step 814, the total forces are compared. If the total force captured by the load cells 272, 274, 276, 27 does not change, step 818 is executed and it is determined that all the data signals being captured are coming from the patient even if the forces captured by the pressure sensors 222, 224, 226, 228, 230 change. This occurrence is processed by the control circuitry 98 as patient movement which may be communicated to the graphical display screen 146 via the control circuitry 98. However, if the total force captured by the load cells 272, 274, 276, 278 shows a sudden increase, step 816 is executed and it is determined by the control circuitry 98 that the data signals being captured are not all coming from the patient and may be communicated to the graphical display screen 146 via the control circuitry 98. At any given time, the caregiver invokes the comparison of the signals being measured by the load cells 272, 274, 276, 278 and pressure sensors 222, 224, 226, 228, 230 to determine their source.

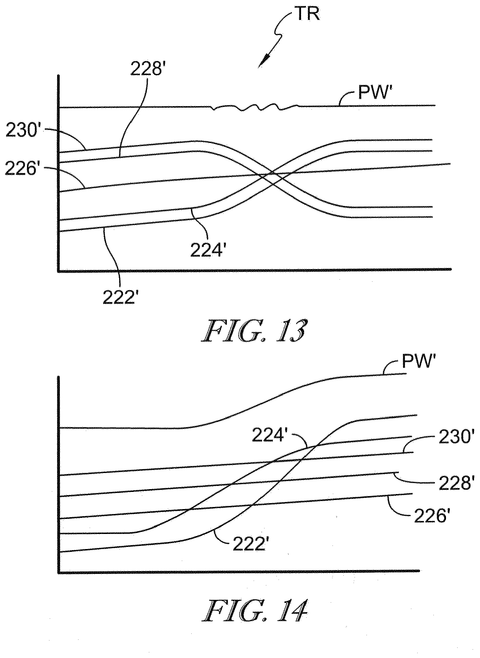

The basis for this determination can be illustrated with regard to the differences between the case shown in Figs. 13 and the case shown in Fig. 14. Fig. 13 shows the sensor response of the pressure sensors 222, 224, 226, 228, 230 as compared to the change in the patient weight (pw). The signal response of the pressure sensors 222, 224, 226, 228, 230 are respectively shown over time with reference to respective signal traces 222', 224', 226', 228', and 230'. The response of the patient weight is indicated by PW. This response curve in Fig. 13 is related to movement of a patient over the bed which results in changes in the pressures in various zones as indicated by the signal traces 222', 224', 226', 228', and 230' where the pressures 222' and 224' increase while pressures 228' and 230' decrease. The concern is whether the changes in pressure are related to patient movement or an extraneous force or addition of weight. However, the signal trace of the patient weight PW' indicates that there was some disruption of the measured patient weight at time range TR, but the overall weight stayed constant. Thus, the control circuitry 98 can discern that the event was patient movement that can be used to calculate patient mobility. This can be contrasted to the case in Fig. 14 which shows that both pressure signal traces 222' and 224' increased and patient weight PW' increased with no offloading of pressures as measured by the other sensors 226, 228, and 230. This is discerned by the control circuitry 98 to be a non-patient event and the related data is not considered in the patient mobility analysis.

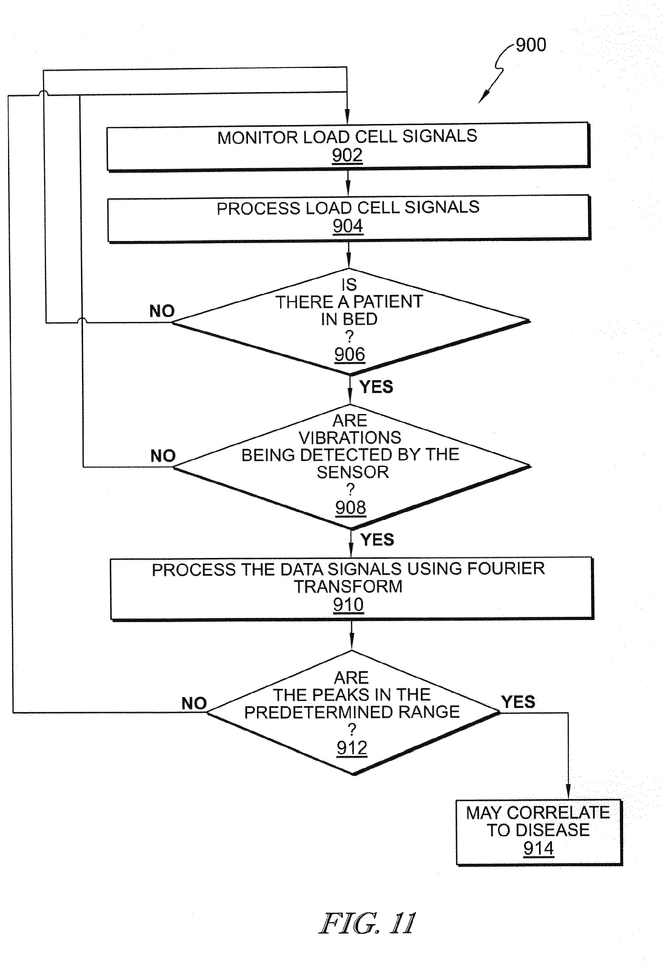

Fig. 11 illustrates a process 900 wherein the control circuitry 98 utilizes the signals from each load cell 272, 274, 276, 278 to detect the vibrations associated with the patient. At process step 902, the signal from each load cell 272, 274, 276, 278 is monitored. At process step 904, the processor module 172 processes the signals from the load cells 272, 274, 276, 278 and stores the signals in the memory 174. The loads detected by each of the respective load cells 272, 274, 276, 278 is adjusted using a corresponding calibration constant. The adjusted loads are then combined to establish the actual weight supported on the patient support apparatus 10. At decision step 906, the actual weight is compared to initial weights stored for that specific patient support apparatus in the memory unit 174 to determine if a patient is present. If there is no patient present, step 902 is repeated. If a patient is present, decision step 908 is executed. At decision step 908, load cell 272, 274, 276, 278 are each respectively monitored to determine if low frequency signal variations are being detected by any one or more of the load cells 272, 274, 276, 278, if so the system determines that the signals should be analyzed for potential vibrations or tremors being experienced by the patient. If tremors are not indicated, step 902 is repeated. If vibrations are indicated, step 910 is executed, where these variations are processed by using a Fourier transform to determine the peaks of the signal variations in the frequency domain. If the frequency domain indicates that the vibrations are determined to be within a predetermined range at decision step 912, the resulting vibrations are processed as an indicator of the onset of a disease, such as Parkinson's disease or epileptic seizure. For example, a signal that falls in the 3 Hz -6 Hz range in the frequency domain may be logged by the control circuitry 98 as an indication of Parkinson's disease at process step 914. This may result in an alert being communicated to a caregiver, such as through the graphical display screen 148 via the control circuitry 98. If vibrations are not detected in a predetermined range, step 902 is repeated.

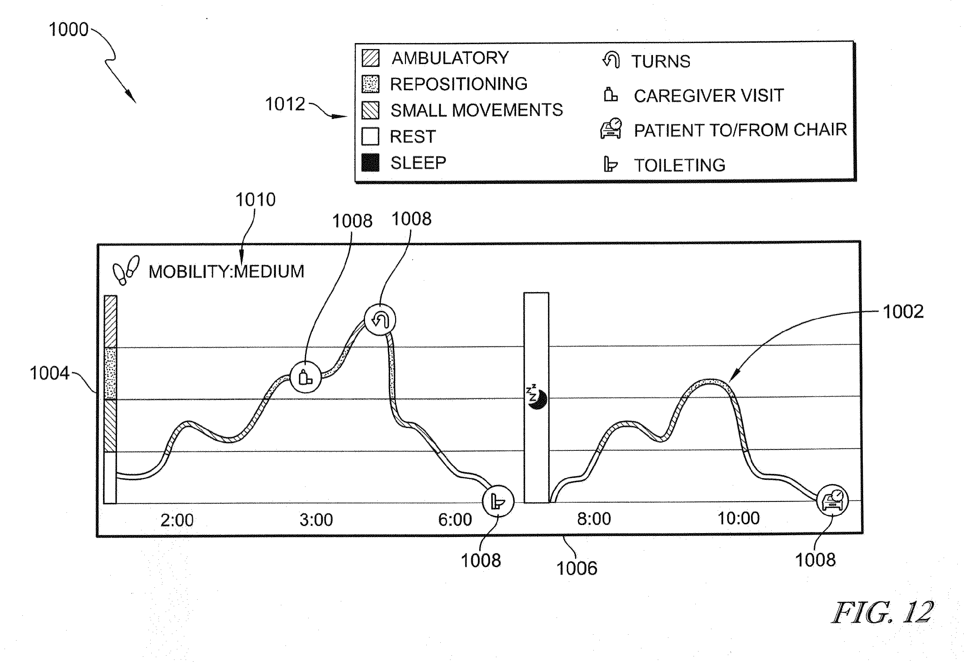

Referring to Fig. 12, a graph 1000 may be generated to display patient movement as a curve 1002 of mobility 1004 over time 1006. The graph 1000 is color coded to display a degree of movement. The variations in cross-hatching shown in the elements of the graph 1000 in Fig. 12 provide an indication of the degree of movement. For example, parts of the curve 1002 may be shown in red to illustrate rest, parts of the curve 1002 may be orange to illustrate small movements, parts of the curve 1002 may be yellow to illustrate repositioning, and parts of the curve 1002 may be green to illustrate that the patient is ambulatory. As will be appreciated, any color scheme may be used on the graph 1000. Graph 1000 provides a time phased graph of mobility, along with important information about the patient's activity. For example, the vertical scale shows the current numerical level of mobility on a four point scale. Indicia 1010 provides an indication of the qualitative level of mobility. The key 1012 provides a user with an ability to interpret the chart and infer certain information about the patient. This information may be provided to systems connected to the bed 10 as well.

In some embodiments, the control circuitry 98 of the bed 10 communicates with a remote computer device 176 shown in Fig. 2 via a communication infrastructure 178 such as an Ethernet of a healthcare facility in which the bed 10 is located and via communications links 177, 179. The computer device 176 is sometimes simply referred to as a "computer" herein. Computer 176 may be part of an electronic medical records (EMR) system, for example. However, it is within the scope of this disclosure for the control circuitry 98 of the bed 10 to communicate with other computers such as those included as part of a nurse call system, a physician ordering system, an admission/discharge/transfer (ADT) system, or some other system used in a healthcare facility in other embodiments. The Ethernet 178 in Fig. 2 is illustrated diagrammatically and is intended to represent all of the hardware and software that comprises a network of a healthcare facility.

The bed 10 has a communication interface or port 180 which provides bidirectional communication via link 179 with the infrastructure 178 which, in turn, communicates bidirectionally with the computer 176 via the link 177. The link 179 is a wired communication link in some embodiments and is a wireless communications link in other embodiments. Using these communication systems, data and analysis performed by the control circuitry 98 may be communicated to external systems.

**Claims**

1. A patient support apparatus (10) comprising:

   a frame (20),
   a patient support surface (22) positioned on the frame (20),
   a plurality of force sensors (222, 224, 226, 228, 230) coupled to at least one of the frame (20) and the patient support surface (22), each of the plurality of force sensors (222, 224, 226, 228, 230) configured to detect a force applied to the respective force sensor (222, 224, 226, 228, 230), and
   a controller (98) in communication with each of the plurality of force sensors, the controller comprising a processor (172) and a memory device (174), wherein the memory device (174) includes instructions that, when executed by the processor (172), cause the processor to process data signals from the plurality of force sensors to determine a mobility score of a patient supported on the patient support surface (22), wherein determining the mobility score includes executing the instructions with the processor (172) to cause the processor to:

      determine a change of position of a center of gravity of the patient,
      multiply the change of position of the center of gravity by a patient weight to compute an instantaneous work,
      sum the instantaneous work over a time period to compute a power, and
      utilize the power as an input to a statistical model to generate the mobility score.

2. The patient support apparatus (10) of claim 1, wherein the mobility score is output to a user.

3. The patient support apparatus (10) of claim 2, wherein the mobility score is output to a display (142) of a user interface.

4. The patient support apparatus (10) of any preceding claim, wherein the data signals from the plurality of force sensors (222, 224, 226, 228, 230) are processed in real-time.

5. The patient support apparatus (10) of any preceding claim, wherein the memory device further includes instructions that, when executed by the processor, cause the processor to store the mobility score over time.

6. The patient support apparatus (10) of claim 5, wherein the memory device further includes instructions that, when executed by the processor, cause the processor to create a data array of the mobility score stored over time.

7. The patient support apparatus (10) of claim 6, wherein the memory device further includes instructions that, when executed by the processor, cause the processor to display at least a portion of the data array as a graph.

8. The patient support apparatus (10) of claim 7, wherein the memory device further includes instructions that, when executed by the processor, cause the processor to display the graph on a display (142) of a user interface.

9. The patient support apparatus (10) of any one of claims 5 to 8, wherein the mobility score over time includes a number of data points.

10. The patient support apparatus (10) of claim 9, wherein each of the data points is selectable on the user interface to display additional information related to the respective data point.

11. The patient support apparatus (10) of claim 10, wherein the additional information includes a summary of data accumulated to a point in time corresponding with the data point.

12. The patient support apparatus (10) of either claim 10 or claim 11, wherein the additional information includes a graphical indication of a time at which the patient moved relative to the patient support surface.

13. The patient support apparatus (10) of any preceding claim, wherein the mobility score includes a plurality of mobility indices.

14. The patient support apparatus (10) of claim 13, wherein each of the mobility indices is modified by at least one predetermined factor.

15. The patient support apparatus (10) of claim 14, wherein the at least one predetermined factor includes at least one of a patient factor, a bed factor, and a statistical scaling factor.

**Patentansprüche**

1. Patientenunterstützungsvorrichtung (10), die Folgendes aufweist:

ein Gestell (20),
eine Patientenauflagefläche (22), die auf dem Gestell (20) positioniert ist,
eine Vielzahl von Kraftaufnehmern (222, 224, 226, 228, 230), die mit dem Gestell (20) und/oder der Patientenauflagefläche (22) gekoppelt sind, wobei jeder der Vielzahl von Kraftaufnehmern (222, 224, 226, 228, 230) zum Erfassen einer auf den jeweiligen Kraftaufnehmer (222, 224, 226, 228, 230) angewendeten Kraft konfiguriert ist, und
eine Steuerung (98), die mit jeder der Vielzahl von Kraftaufnehmern in Kommunikation steht, wobei die Steuerung einen Prozessor (172) und eine Speichervorrichtung (174) aufweist, wobei die Speichervorrichtung (174) Anweisungen beinhaltet, die bei Ausführung durch den Prozessor (172) den Prozessor zum Verarbeiten von Datensignalen von der Vielzahl von Kraftaufnehmern zum Bestimmen einer Mobilitätsbewertung eines auf der Patientenauflagefläche (22) gelagerten Patienten veranlassen, wobei das Bestimmen der Mobilitätsbewertung das Ausführen der Anweisungen mit dem Prozessor (172) beinhaltet zum Veranlassen des Prozessors zum:

Bestimmen einer Änderung der Position eines Schwerpunkts des Patienten,
Multiplizieren der Änderung der Position des Schwerpunkts mit einem Patientengewicht zum Berechnen einer momentanen Arbeit,
Summieren der momentanen Arbeit über eine Zeitdauer zum Berechnen einer Leistung und
Nutzen der Leistung als Eingabe in ein statistisches Modell zur Erzeugung der Mobilitätsbewertung.

2. Patientenunterstützungsvorrichtung (10) nach Anspruch 1, wobei die Mobilitätsbewertung an einen Benutzer ausgegeben wird.

3. Patientenunterstützungsvorrichtung (10) nach Anspruch 2, wobei die Mobilitätsbewertung an eine Anzeige (142) einer Benutzeroberfläche ausgegeben wird.

4. Patientenunterstützungsvorrichtung (10) nach einem der vorhergehenden Ansprüche, wobei die Datensignale von der Vielzahl von Kraftaufnehmern (222, 224, 226, 228, 230) in Echtzeit verarbeitet werden.

5. Patientenunterstützungsvorrichtung (10) nach einem der vorhergehenden Ansprüche, wobei die Speichervorrichtung ferner Anweisungen beinhaltet, die bei Ausführung durch den Prozessor den Prozessor zum Speichern der Mobilitätsbewertung im Zeitverlauf veranlassen.

6. Patientenunterstützungsvorrichtung (10) nach Anspruch 5, wobei die Speichervorrichtung ferner Anweisungen beinhaltet, die bei Ausführung durch den Prozessor den Prozessor zum Erstellen eines Datenfelds der im Zeitverlauf gespeicherten Mobilitätsbewertung veranlassen.

7. Patientenunterstützungsvorrichtung (10) nach Anspruch 6, wobei die Speichervorrichtung ferner Anweisungen beinhaltet, die bei Ausführung durch den Prozessor den Prozessor zum Anzeigen wenigstens eines Teils des Datenfelds als ein Diagramm veranlassen.

8. Patientenunterstützungsvorrichtung (10) nach Anspruch 7, wobei die Speichervorrichtung ferner Anweisungen beinhaltet, die bei Ausführung durch den Prozessor den Prozessor zum Anzeigen des Diagramms auf einer Anzeige (142) einer Benutzeroberfläche veranlassen.

9. Patientenunterstützungsvorrichtung (10) nach einem der Ansprüche 5 bis 8, wobei die Mobilitätsbewertung im Zeitverlauf eine Anzahl von Datenpunkten beinhaltet.

10. Patientenunterstützungsvorrichtung (10) nach Anspruch 9, wobei jeder der Datenpunkte auf der Benutzeroberfläche auswählbar ist, um zusätzliche Informationen in Bezug auf den jeweiligen Datenpunkt anzuzeigen.

11. Patientenunterstützungsvorrichtung (10) nach Anspruch 10, wobei die zusätzlichen Informationen eine Zusammenfassung von bis zu einem dem Datenpunkt entsprechenden Zeitpunkt angesammelten Daten beinhalten.

12. Patientenunterstützungsvorrichtung (10) nach Anspruch 10 oder Anspruch 11, wobei die zusätzlichen Informationen eine graphische Angabe zu einer Zeit, zu der der Patient sich relativ zur Patientenauflagefläche bewegte, beinhalten.

13. Patientenunterstützungsvorrichtung (10) nach einem der vorhergehenden Ansprüche, wobei die Mobilitätsbewertung eine Vielzahl von Mobilitätsindizes beinhaltet.

14. Patientenunterstützungsvorrichtung (10) nach Anspruch 13, wobei die Mobilitätsindizes jeweils um wenigstens einen vorbestimmten Faktor modifiziert sind.

15. Patientenunterstützungsvorrichtung (10) nach Anspruch 14, wobei der wenigstens eine vorbestimmte Faktor wenigstens einen von einem Patientenfaktor, einem Bettfaktor und einem statistischen Skalierungsfaktor beinhaltet.

## Revendications

1. Appareil de support de patient (10) comprenant :

un cadre (20),
une surface de support de patient (22) positionnée sur le cadre (20),
une pluralité de capteurs de force (222, 224, 226, 228,230) couplés au au moins un cadre (20) et à la surface de support de patient (22), chacun de la pluralité de capteurs de force (222, 224, 226, 228,230) étant configuré pour détecter une force appliquée au capteur de force respectif (222, 224, 226, 228,230), et
un contrôleur (98) en communication avec chacun de la pluralité de capteurs de force, le contrôleur comprenant un processeur (172) et un dispositif de mémoire (174), dans lequel le dispositif de mémoire (174) comprend des instructions qui, lorsque exécutées par le processeur (172), font que le processeur traite des signaux de données de la pluralité de capteurs de force afin de déterminer un score de mobilité d'un patient soutenu sur la surface de support de patient (22), dans lequel déterminer le score de mobilité comprend exécuter les instructions avec le processeur (172) pour faire que le processeur :

détermine un changement de position d'un centre de gravité du patient,

multiplie le changement de position du centre de gravité par un poids du patient pour calculer un travail instantané,

totalise le travail instantané au cours d'une période de temps pour calculer une puissance, et

utilise la puissance comme une entrée dans un modèle statistique pour générer le score de mobilité.

2. Appareil de support de patient (10) selon la revendication 1, dans lequel le score de mobilité est sorti à un utilisateur.

3. Appareil de support de patient (10) selon la revendication 2, dans lequel le score de mobilité est sorti à un affichage (142) d'une interface utilisateur.

4. Appareil de support de patient (10) selon l'une quelconque des revendications précédentes, dans lequel les signaux de données de la pluralité de capteurs de force (222, 224, 226, 228, 230) sont traités en temps réel.

5. Appareil de support de patient (10) selon l'une quelconque des revendications précédentes, dans lequel le dispositif de mémoire comprend en outre des instructions qui, lorsque exécutées par le processeur, font que le processeur stocke le score de mobilité au cours du temps.

6. Appareil de support de patient (10) selon la revendication 5, dans lequel le dispositif de mémoire comprend en outre des instructions qui, lorsque exécutées par le processeur, font que le processeur crée un ensemble de données du score de mobilité stocké au cours du temps.

7. Appareil de support de patient (10) selon la revendication 6, dans lequel le dispositif de mémoire comprend en outre des instructions qui, lorsque exécutées par le processeur, font que le processeur affiche au moins une partie de l'ensemble de données comme un graphique.

8. Appareil de support de patient (10) selon la revendication 7, dans lequel le dispositif de mémoire comprend en outre des instructions qui, lorsque exécutées par le processeur, font que le processeur affiche le graphique sur un affichage (142) d'une interface utilisateur.

9. Appareil de support de patient (10) selon l'une quelconque des revendications 5 à 8, dans lequel le score de mobilité au cours du temps comprend un nombre de points de données.

10. Appareil de support de patient (10) selon la revendication 9, dans lequel chacun des points de données est sélectionnable sur l'interface utilisateur afin d'afficher des informations supplémentaires concernant le point de données respectif.

11. Appareil de support de patient (10) selon la revendication 10, dans lequel les informations supplémentaires comprennent un résumé de données accumulées à un point dans le temps correspondant au point de données.

12. Appareil de support de patient (10) selon la revendication 10 ou la revendication 11, dans lequel les informations supplémentaires comprennent une indication graphique d'un moment auquel le patient a bougé par rapport à la surface de support de patient.

13. Appareil de support de patient (10) selon l'une quelconque des revendications précédentes, dans lequel le score de mobilité comprend une pluralité d'indices de mobilité.

14. Appareil de support de patient (10) selon la revendication 13, dans lequel chacun des indices de mobilité est modifié par au moins un facteur prédéterminé.

15. Appareil de support de patient (10) selon la revendication 14, dans lequel le au moins un facteur prédéterminé comprend au moins l'un d'entre un facteur de patient, un facteur de lit et un facteur d'échelle statistique.

*FIG. 1*

*FIG. 2*

FIG. 3

EP 3 912 609 B1

*FIG. 4*

*FIG. 5*

*FIG. 6*

*FIG. 7*

331

352

350 350

350

348

4
3
2
1

346

344

302

2 HRS. 01/09/12 2:59
4 HRS. 01/09/12 4:59
6 HRS. 01/09/12 6:59
8 HRS. 01/09/12 8:59
10 HRS. 01/09/12 10:59
12 HRS. 01/09/12 12:59
14 HRS. 01/09/12 14:59
16 HRS. 01/09/12 16:59
18 HRS. 01/09/12 18:59
20 HRS. 01/09/12 20:59

CURRENT STATUS: | MODERATE | 334

TURN INTERVAL: | 2 HRS | 338

TIME SINCE LAST TURN: | 6 HRS. 15 MINS. | 342

332

336

340

FIG. 8

302

TURN MONITORING: [ ADD BASELINE ]  335

332 — CURRENT STATUS: [ SUPINE ]  334

336 — TURN INTERVAL: [ 2 HRS ]  338

340 — TIME SINCE LAST TURN: [ 6 HRS. 15 MINS. ]  342

*FIG. 9*

EP 3 912 609 B1

800

```
┌─────────────────────────────────────────┐
│      MONITOR LOAD CELL SIGNALS           │
│                 802                      │
└─────────────────────────────────────────┘
                    │
                    ▼
┌─────────────────────────────────────────┐
│      PROCESS LOAD CELL SIGNALS           │
│                 804                      │
└─────────────────────────────────────────┘
                    │
                    ▼
              IS
         THERE A PATIENT
    NO       IN BED
              ?
             806
                    │
                    ▼
┌─────────────────────────────────────────┐
│   RECEIVE SIGNALS FROM PRESSURE SENSOR   │
│                 808                      │
└─────────────────────────────────────────┘
                    │
                    ▼
┌─────────────────────────────────────────┐
│   RECEIVE SIGNALS FROM LOAD CELLS ON THE │
│   PATIENT SUPPORT ASSEMBLY PERIMETER     │
│                 810                      │
└─────────────────────────────────────────┘
                    │
                    ▼
┌─────────────────────────────────────────┐
│ PROCESS THE DATA SIGNALS FROM THE PRESSURE│
│ SENSORS AND LOAD CELLS IN TANDEM TO DETERMINE│
│ IF THE PATIENT OR ANOTHER PERSON IS IMPARTING│
│      THE SIGNALS BEING MEASURED          │
│                 812                      │
└─────────────────────────────────────────┘
                    │
                    ▼
               DOES
          THE TOTAL FORCE
   YES  MEASURED BY LOAD CELLS   NO
             CHANGE
               ?
              814
```

OUTPUT - CHANGE IS DUE LOAD INDEPENDENT OF THE PATIENT
816

OUTPUT - CHANGE IS DUE MOVEMENT BY THE PATIENT
818

*FIG. 10*

*FIG. 11*

**1000**

Legend (**1012**):
- ▨ AMBULATORY
- ▦ REPOSITIONING
- ▨ SMALL MOVEMENTS
- ☐ REST
- ■ SLEEP
- ↰ TURNS
- CAREGIVER VISIT
- PATIENT TO/FROM CHAIR
- TOILETING

MOBILITY:MEDIUM

2:00    3:00    6:00    8:00    10:00

FIG. 12

EP 3 912 609 B1

*FIG. 13*

*FIG. 14*

**EP 3 912 609 B1**

**REFERENCES CITED IN THE DESCRIPTION**

**Patent documents cited in the description**

- WO 2016073186 A1
- US 10634549 B
- US 10054479 B